(19) Europäisches Patentamt
European Patent Office
Office européen des brevets

(11) **EP 1 600 148 B1**

(12) **FASCICULE DE BREVET EUROPEEN**

(45) Date de publication et mention
de la délivrance du brevet:
**20.02.2008 Bulletin 2008/08**

(51) Int Cl.:
*A61K 8/41* *(2006.01)*  *A61K 8/81* *(2006.01)*
*A61K 8/73* *(2006.01)*  *A61K 8/40* *(2006.01)*
*A61Q 5/10* *(2006.01)*

(21) Numéro de dépôt: **05291134.4**

(22) Date de dépôt: **26.05.2005**

(54) **Composition pour la coloration des fibres kératiniques comprenant un composé portant au moins une fonction amine, un pigment et un agent de couplage chimique**

Mittel zur Färbung von keratinischen Fasern enthaltend einen aminofunktionalisierten Agentien, ein Pigment und einen chemischen Kuppler

Composition for dyeing keratinous fibres and containing at least one amine-functionalised compound, one pigment and one chemical coupling agent

(84) Etats contractants désignés:
**AT BE BG CH CY CZ DE DK EE ES FI FR GB GR HU IE IS IT LI LT LU MC NL PL PT RO SE SI SK TR**

(30) Priorité: **28.05.2004 FR 0405842**

(43) Date de publication de la demande:
**30.11.2005 Bulletin 2005/48**

(73) Titulaire: **L'ORÉAL**
**75008 Paris (FR)**

(72) Inventeur: **Brun, Gaelle**
**75015 Paris (FR)**

(74) Mandataire: **Fevrier, Murielle Françoise E.**
**L'OREAL - D.I.P.I.**
**25-29 Quai Aulagnier**
**92600 Asnières (FR)**

(56) Documents cités:
WO-A-02/13773          DE-A- 19 847 883
FR-A- 1 567 219

# EP 1 600 148 B1

## Description

La présente invention a pour objet une composition pour la coloration des fibres kératiniques, et en particulier des fibres kératiniques humaines telles que les cheveux, comprenant au moins un composé portant au moins une fonction amine, au moins un pigment différent de l'oxyde de fer, l'oxyde de titane et la silice, et au moins un agent de couplage chimique pour la formation de liaisons amide.

Dans le domaine de la teinture des fibres kératiniques, il existe deux modes de coloration qui présentent chacun leurs avantages et leurs inconvénients.

La *coloration directe* ou *coloration semi-permanente* consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique azinique, triarylméthane ou des colorants naturels.

Il a par ailleurs déjà été proposé l'utilisation de pigments, comme dans la demande de brevet FR 2 741 530, qui préconise l'utilisation pour la coloration des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient d'avoir une faible résistance aux shampooings.

La *coloration d'oxydation* ou *coloration permanente* consiste à teindre les fibres kératiniques avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

Les nuances obtenues avec la coloration d'oxydation présentent en général une assez bonne ténacité aux shampooings.

La difficulté avec ces deux modes de coloration, la *coloration directe* ou *coloration semi-permanente* d'une part, et la *coloration d'oxydation* ou *coloration permanente* d'autre part, est que si l'on souhaite colorer un support foncé, il est nécessaire de décolorer ou d'éclaircir le support afin que la couleur apportée soit visible.

La décoloration ou l'éclaircissement des fibres kératiniques peut être réalisé à l'aide d'un agent oxydant. Parmi les agents oxydants classiquement utilisés, on peut citer le peroxyde d'hydrogène ou des composés susceptibles de produire du peroxyde d'hydrogène par hydrolyse, comme par exemple le peroxyde d'urée ou les persels tels que les perborates, les persulfates, les percarbonates.

L'utilisation d'agents oxydants, que ce soit pour éclaircir et / ou pour oxyder les colorants d'oxydation, présente l'inconvénient d'entraîner une dégradation non négligeable des fibres kératiniques et d'altérer leurs propriétés cosmétiques. Les cheveux ont tendance à devenir rèches, plus difficilement démêlables et plus fragiles.

Le but de la présente invention est de fournir de nouvelles compositions pour la coloration des fibres kératiniques, et en particulier des fibres kératiniques humaines telles que les cheveux, qui permettent d'obtenir des colorations qui sont visibles sur support foncé sans qu'il soit nécessaire d'éclaircir ou de décolorer les fibres et qui présentent une bonne résistance aux shampooings.

Ce but est atteint avec la présente invention qui a pour objet une composition pour la coloration des fibres kératiniques comprenant, dans un milieu cosmétiquement acceptable :

- au moins un composé portant au moins une fonction amine et ayant un poids moléculaire supérieur à 300 g.mol$^{-1}$;
- au moins un pigment différent de l'oxyde de fer, de l'oxyde de titane et de la silice ; et
- au moins un agent de couplage chimique pour la formation de liaisons amide.

La composition conforme à la présente invention conduit à une coloration visible sur support foncé sans qu'il soit nécessaire d'éclaircir ou de décolorer les fibres kératiniques, et par conséquent sans dégradation physique des fibres kératiniques. Cette coloration est de plus résistante aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis à vis des shampooings et des frottements. La coloration est obtenue dans des nuances variées, elle est chromatique, puissante, esthétique et peu sélective.

La présente invention a aussi pour objet un procédé de coloration des fibres kératiniques mettant en oeuvre au moins un composé portant au moins une fonction amine et ayant un poids moléculaire supérieur à 300 g.mol$^{-1}$; au moins un pigment différent de l'oxyde de fer, l'oxyde de titane et la silice, et au moins un agent de couplage chimique pour la formation de liaisons amide.

Un autre objet de la présente invention est l'utilisation pour la coloration des fibres kératiniques d'au moins un composé portant au moins une fonction amine et ayant un poids moléculaire supérieur à 300 g.mol$^{-1}$, d'au moins un pigment différent de l'oxyde de fer, l'oxyde de titane et la silice, et d'au moins un agent de couplage chimique pour la formation de liaisons amide.

Le ou les composés portant au moins une fonction amine ont un poids moléculaire supérieur à 300 g.mol$^{-1}$. De préférence, le ou les composés portant au moins une fonction amine ont un poids moléculaire supérieur à 1000 g.mol$^{-1}$. A titre d'exemples, on peut citer les polymères.

Parmi les polymères portant au moins une fonction amine, on préfère les polyamines. A titre d'exemples de polyamines, on peut citer la polyvinylamine, la polyéthylèneamine, la polylysine, le chitosane, la polyallylamine, l'aminodextrane, l'amino cellulose, l'aminopolyvinylacétal.

Par pigment, on entend toute entité organique et / ou minérale dont la solubilité dans l'eau est inférieure à 0,01 % à 20 °C, présentant une absorption entre 350 et 700 nm, de préférence une absorption avec un maximum.

Les pigments conformes à la présente invention sont choisis parmi tous les pigments organiques et / ou minéraux, à l'exception de l'oxyde de fer, de l'oxyde de titane et de la silice, notamment ceux qui sont décrits dans l'encyclopédie de technologie chimique de Kirk-Othmer et dans l'encyclopédie de chimie industrielle de Ullmann.

Les pigments conformes à l'invention peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer les oxydes de zirconium ou de cérium, les oxydes de chrome, le violet de manganèse, le bleu outremer, l'hydrate de chrome et le bleu ferrique.

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom :

- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant :

- un noyau inorganique,
- au moins un liant assurant la fixation des pigments organiques sur le noyau, et

- au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriquées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of physical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

Les pigments conformes à l'invention sont de préférence des pigments colorés.

La variété des pigments mis en jeu permet d'obtenir une riche palette de couleurs, ainsi que des effets optiques particuliers tels que des effets métalliques, interférentiels.

La taille d'un pigment autre que les nacres en solution est généralement comprise entre 10 nm et 10 $\mu$m, de préférence entre 50 nm et 5 $\mu$m, et encore plus préférentiellement entre 100 nm et 3 $\mu$m. La taille d'une nacre en solution est généralement comprise entre 1 et 200 $\mu$m, de préférence entre 1 et 80 $\mu$m, et encore plus préférentiellement entre 1 et 50 $\mu$m.

Selon un mode de réalisation particulier de l'invention, le ou les pigments sont en dispersion dans la composition conforme à l'invention.

Dans le cadre de la présente invention, on entend par agent de couplage chimique un composé chimique capable d'augmenter la vitesse d'une réaction. En particulier, un agent de couplage chimique pour la formation de liaisons amide est un composé chimique capable de promouvoir ou d'accélérer la formation de liaisons amide entre un ou plusieurs composés. On peut par exemple citer les dérivés du carbodiimide ; le 1,1'-carbonyldiimidazole ; les sels de phosphonium ; les phosphonates ; les phosphoramides ; les dérivés de dialcoxytriazine tels que le chlorure de 4-(4,6-diméthoxy-1,3,5-triazin-2-yl)-4-méthylmorpholinium (DMT-MM) et le 2-chloro-4,6-diméthoxy-1,3,5-triazine (CDMT).

Selon un mode de réalisation particulier de l'invention, le ou les dérivés du carbodiimide sont choisis parmi les composés de formule (I) suivante :

$$R_1\text{-}N\text{=}C\text{=}N\text{-}R_2 \qquad (I)$$

dans laquelle $R_1$ et $R_2$ désignent, indépendamment l'un de l'autre :

- un atome d'hydrogène ;
- un radical alkyle non substitué ou substitué par un ou plusieurs substituants choisis parmi :

- un radical hydroxyle ;
- un radical amino ;
- un radical mono ou dialkylamino ;
- un radical mono ou di(mono ou polyhydroxyalkyl)amino ;
- un radical aryle ;
- un groupement hétérocyclique ;

- un radical aryle ;
- un radical arylalkyle.

Dans le cadre de la présente invention, on entend par radical alkyle un radical linéaire ou ramifié, cyclique ou acyclique, comprenant de 1 à 30 atomes de carbone, de préférence de 1 à 10 atomes de carbone, par exemple les radicaux méthyle, éthyle, n-propyle, iso-propyle, butyle.

On entend par radical aryle un radical carboné dérivé des composés benzéniques condensés ou non condensés, comprenant de 6 à 30 atomes de carbone, par exemple les radicaux phényle, anthracényle et naphtyle.

On entend par groupement hétérocyclique un groupement mono ou polycyclique, condensé ou non condensé, aromatique ou non aromatique, comprenant de 5 à 50 chaînons, de préférence de 5 à 10 chaînons, par exemple un cycle thiophène, benzofurane, benzothiophène, indole, bispyridine, benzopyrane, quinoline, pyrazole, pyridine, pyrrole, furane, imidazole, benzimidazole.

De préférence, $R_1$ et $R_2$ ne désignent pas un atome hydrogène. Avantageusement, $R_1$ et $R_2$ désignent un radical alkyle non substitué ou substitué.

A titre d'exemples des dérivés de carbodiimide utiles dans le cadre de l'invention, on peut citer le N-(3-diméthylaminopropyl) N'-(éthyl) carbodiimide ; le N,N'-dicyclohexylcarbodiimide.

De préférence, le ou les dérivés du carbodiimide sont solubles dans l'eau ou dans un solvant alcoolique.

Le ou les agents de couplage chimiques peuvent être supportés par un polymère. Par exemple, le chlorhydrate de N-(3-diméthylaminopropyl)-N'-(éthyl) carbodiimide (EDC) supporté par un polymère avec un taux de fonctionnalisation de 1,4 mmol d'EDC par gramme de polymère est commercialisé par Fluka (code 09657).

Le ou les composés portant au moins une fonction amine et ayant un poids moléculaire supérieur à 300 g.mol$^{-1}$ sont chacun généralement présents dans la composition conforme à l'invention à des concentrations comprises entre 0,1 et 60 % en poids, de préférence entre 0,2 et 40 % en poids, et encore plus préférentiellement entre 0,5 et 35 % en poids par rapport au poids total de la composition.

Le ou les pigments sont chacun généralement présents dans la composition conforme à l'invention à des concentrations comprises entre 0,05 et 80 % en poids, de préférence entre 0,1 et 60 % en poids, et encore plus préférentiellement entre 0,25 et 40 % en poids par rapport au poids total de la composition.

Le ou les agents de couplage chimiques sont chacun généralement présents dans la composition conforme à l'invention à des concentrations comprises entre 0,05 et 30 % en poids, de préférence entre 0,1 et 20 % en poids, et encore plus préférentiellement entre 0,25 et 10 % en poids par rapport au poids total de la composition.

La composition selon la présente invention peut de plus comprendre au moins un composé additionnel qui, en association avec le ou les agents de couplage chimiques, sont capables de favoriser la formation de liaisons amide.

Selon un mode de réalisation particulier de l'invention, le ou les composés additionnels sont des composés portant au moins une fonction N-hydroxy.

A titre d'exemples de composés portant au moins une fonction N-hydroxy, on peut citer le N-hydroxysuccinimide ; le N-hydroxysulfosuccinimide.

De préférence, le ou les composés portant au moins une fonction N-hydroxy contiennent de plus au moins un groupement polaire.

Le ou les composés additionnels qui, en association avec le ou les agents de couplage chimiques, sont capables de favoriser la formation de liaisons amide sont chacun généralement présents à des concentrations comprises entre 0,05 et 30 % en poids, de préférence entre 0,1 et 20 % en poids, et encore plus préférentiellement entre 0,25 et 10 % en poids par rapport au poids total de la composition.

La composition conforme à l'invention peut de plus comprendre des charges, à l'exception de la silice.

Par charges, il faut comprendre des particules incolores ou blanches, minérales ou de synthèse, lamellaires ou non lamellaires. Les charges peuvent être présentes à raison de 0 à 80 % en poids, par rapport au poids total de la composition, de préférence de 0,01 à 60 % en poids, et mieux de 0,02 % à 40 % en poids. On peut notamment citer le talc, le stéarate de zinc, le kaolin, les poudres de polyamide (Nylon®) (Orgasol de chez Atochem), les poudres de polyéthylène, les poudres de polymères de tétrafluoroéthylène (TéflonB), l'amidon, le nitrure de bore, des microsphères polymériques telles que celles de chlorure de polyvinylidène / acrylonitrile comme l'Expancel (Nobel Industrie), de copolymères d'acide acrylique (PolytrapB de la société Dow Corning) et les microbilles de résine de silicone (TospearlsO de Toshiba, par exemple), les organopolysiloxanes élastomères.

La composition selon l'invention contient avantageusement, en outre, des additifs cosmétiques conventionnels choisis parmi les polymères fixants, les épaississants, les tensioactifs anioniques, non ioniques, cationiques ou amphotères, les colorants d'oxydation tels que les bases d'oxydation et les coupleurs, les colorants directs autres que les pigments, les parfums, les conservateurs, les agents conditionneurs, les filtres solaires, les protéines, les vitamines, les provitamines, les polymères non fixants anioniques, non ioniques, cationiques ou amphotères, les huiles minérales, végétales ou synthétiques, les céramides, les pseudocéramides, les silicones volatiles ou non, linéaires
ou cycliques, modifiées ou non, les agents régulateurs de pH, les oxydants, les réducteurs, les inhibiteurs, les catalyseurs et tout autre additif classiquement utilisé dans les compositions cosmétiques.

Le procédé selon la présente invention consiste à appliquer sur les fibres kératiniques :

- au moins un composé portant au moins une fonction amine tel que défini précédemment ;
- au moins un pigment tel que défini précédemment ;
- et au moins un agent de couplage chimique tel que défini précédemment.

Le ou les composés portant au moins une fonction amine et ayant un poids moléculaire supérieur à 300 g.mol$^{-1}$, le ou les pigments et le ou les agents de couplage chimiques peuvent être appliqués sur les fibres kératiniques à partir de plusieurs compositions contenant le ou les composés portant au moins une fonction amine, le ou les pigments et le ou les agents de couplage chimiques, seuls ou en mélange, ou à partir d'une seule composition contenant ledit ou lesdits composés portant au moins une fonction amine, le ou les pigments et le
ou les agents de couplage chimiques.

Selon un mode de réalisation particulier de l'invention, on applique sur les fibres kératiniques une composition (A) comprenant, dans un milieu cosmétiquement acceptable, le ou les composés portant au moins une fonction amine et ayant un poids moléculaire supérieur à 300 g.mol$^{-1}$, le ou les pigments, ainsi que le ou les agents de couplage chimiques.

Selon un autre mode de réalisation particulier de l'invention, on applique sur les fibres kératiniques une composition (B) comprenant, dans un milieu cosmétiquement acceptable, le ou les composés portant au moins une fonction amine et ayant un poids moléculaire supérieur à 300 g.mol$^{-1}$, et une composition (C) comprenant, dans un milieu cosmétiquement acceptable, le ou les pigments et le ou les agents de couplage chimiques, l'ordre d'application des compositions (B) et (C) étant indifférent.

Selon un autre mode de réalisation particulier de l'invention, on applique sur les fibres kératiniques une composition (B) comprenant, dans un milieu cosmétiquement acceptable, le ou les composés portant au moins une fonction amine et ayant un poids moléculaire supérieur à 300 g.mol$^{-1}$, une composition (D) comprenant, dans un milieu cosmétiquement acceptable, le ou les pigments et une composition (E) comprenant, dans un milieu cosmétiquement acceptable, le ou les agents de couplage chimiques, l'ordre d'application des compositions (B), (D) et (E) étant indifférent.

Selon un autre mode de réalisation particulier de l'invention, on applique sur les fibres kératiniques une composition (F) comprenant, dans un milieu cosmétiquement acceptable, le ou les composés portant au moins une fonction amine et ayant un poids moléculaire supérieur à 300 g.mol$^{-1}$ et le ou les agents de couplage chimiques et une composition (D) comprenant, dans un milieu cosmétiquement acceptable, le ou les pigments, l'ordre d'application des compositions (F) et (D) étant indifférent.

Selon un autre mode de réalisation particulier de l'invention, on applique sur les fibres kératiniques une composition (E) comprenant, dans un milieu cosmétiquement acceptable, le ou les agents de couplage chimiques et une composition (G) comprenant, dans un milieu cosmétiquement acceptable, le ou les composés portant au moins une fonction amine et ayant un poids moléculaire supérieur à 300 g.mol$^{-1}$ et le ou les pigments, l'ordre d'application des compositions (E) et (G) étant indifférent.

Selon un mode de réalisation préféré de l'invention, la composition comprenant le ou les pigments est appliquée avant la ou les compositions comprenant le ou les composés portant au moins une fonction amine et ayant un poids moléculaire supérieur à 300 g.mol$^{-1}$ et / ou le ou les agents de couplage chimiques.

Selon un mode de réalisation particulier de l'invention, on applique sur les fibres kératiniques au moins un composé additionnel qui, en association avec le ou les agents de couplage chimiques, est capable de favoriser la formation de liaisons amide, tel que défini précédemment, pour favoriser la formation de liaisons amide.

Par exemple, le ou les composés additionnels peuvent être présents dans l'une ou l'autre ou dans plusieurs des compositions appliquées sur les fibres kératiniques ou dans une composition supplémentaire, auquel cas l'ordre d'application des différentes compositions sur les fibres kératiniques est indifférent.

Selon un mode de réalisation particulier de l'invention, le ou les pigments sont en dispersion dans les différentes compositions mises en oeuvre dans le procédé conforme à l'invention les comprenant.

Dans ces compositions, le ou les composés portant au moins une fonction amine et ayant un poids moléculaire supérieur à 300 g.mol$^{-1}$ sont chacun généralement présents à des concentrations comprises entre 0,1 et 60 % en poids, de préférence entre 0,2 et 40 % en poids, et encore plus préférentiellement entre 0,5 et 35 % en poids par rapport au poids total de la composition les comprenant, le ou les pigments sont chacun généralement présents à des concentrations comprises entre 0,05 et 80 % en poids, de préférence entre 0,1 et 60 % en poids, et encore plus préférentiellement entre 0,25 et 40 % en poids par rapport au poids total de la composition les comprenant, le ou les agents de couplage chimiques sont chacun généralement présents à des concentrations comprises entre 0,05 et 30 % en poids, de préférence entre 0,1 et 20 % en poids, et encore plus préférentiellement entre 0,25 et 10 % en poids par rapport au poids total de la composition les comprenant.

Ces compositions peuvent contenir en outre divers additifs cosmétiques conventionnels tels que définis précédemment.

Les différentes compositions mises en oeuvre dans le procédé conforme à l'invention peuvent être appliquées sur des cheveux secs ou humides.

Un rinçage et / ou un séchage intermédiaire peut être réalisé entre chaque application. La mèche peut être séchée au casque, au sèche cheveux, au fer à lisser.

Selon les mêmes procédés, il est possible de réaliser des superpositions multiples de couches de polymères qui réticulent entre eux pour atteindre le type de dépôt souhaité (en termes de nature chimique, résistance mécanique, épaisseur, aspect, toucher...).

Dans le cadre de la présente invention, un milieu cosmétiquement acceptable comprend généralement de l'eau et / ou un ou plusieurs solvants cosmétiquement acceptables tels que les alcools, les esters, les cétones ou les silicones volatiles cycliques ou des mélanges eau-solvant(s), ces solvants étant de préférence des alcools en $C_1$-$C_4$.

La présente invention a aussi pour objet un dispositif à plusieurs compartiments contenant au moins deux compositions telles que l'ensemble desdites compositions comprend :

- au moins un composé portant au moins une fonction amine tel que défini précédemment ;
- au moins un pigment tel que défini précédemment ;
- et au moins un agent de couplage chimique tel que défini précédemment.

Selon un mode de réalisation particulier de l'invention, un premier compartiment contient la composition (B) telle que définie précédemment et un deuxième compartiment contient la composition (C) telle que définie précédemment.

Selon un autre mode de réalisation particulier, un premier compartiment contient la composition (B) telle que définie précédemment, un deuxième compartiment contient la composition (D) telle que définie précédemment et un troisième compartiment contient la composition (E) telle que définie précédemment.

Selon un autre mode de réalisation particulier de l'invention, un premier compartiment contient la composition (F) telle que définie précédemment et un deuxième compartiment contient la composition (D) telle que définie précédemment.

Selon un autre mode de réalisation particulier, un premier compartiment contient la composition (E) telle que définie précédemment et un deuxième compartiment contient la composition (G) telle que définie précédemment.

Selon un mode de réalisation particulier de l'invention, l'ensemble desdites compositions comprend de plus au moins un composé additionnel qui, en association avec le ou les agents de couplage chimiques, est capable de favoriser la formation de liaisons amide, tel que défini précédemment.

Par exemple, l'une ou l'autre ou plusieurs des compositions contenues dans le dispositif à plusieurs compartiments peut de plus comprendre le ou les composés additionnels.

Le dispositif à plusieurs compartiments peut aussi contenir une composition supplémentaire comprenant, dans un milieu cosmétiquement acceptable, le ou les composés additionnels.

La présente invention a également pour objet l'utilisation pour la coloration des fibres kératiniques d'au moins un composé portant au moins une fonction amine tel que défini précédemment, d'au moins un pigment tel que défini précédemment et d'au moins un agent de couplage chimique tel que défini précédemment.

En particulier, la présente invention a pour objet l'utilisation pour la coloration des fibres kératiniques d'au moins un composé portant au moins une fonction amine tel que défini précédemment, d'au moins un pigment tel que défini précédemment et d'au moins un agent de couplage chimique tel que défini précédemment, dans laquelle la coloration des fibres kératiniques est résistante aux shampooings.

De préférence, la résistance aux shampooings est telle que le pourcentage de dégradation après 6 shampooings tel que défini ci-après est inférieur à 50 %.

L'invention va être plus complètement illustrée à l'aide des exemples non limitatifs suivants.

**EXEMPLES**

Les exemples suivants ont été réalisés en utilisant des mèches de 1 g de cheveux naturels.

Les différents réactifs utilisés sont les suivants :

- Pigment 1 : Pigment composite de 17 nm avec un coeur de silice et une partie organique constituée de quinacridone (pigment Red 122) préparé par la méthode décrite dans la demande de brevet EP 1 184 426 A2 ;
- Pigment 2 : Sel de calcium du rouge lithol B, D&C Red 7 commercialisé par la société Wackherr ;
- EDC : chlorhydrate de N-(3-diméthylaminopropyl)-N'-(éthyl) carbodiimide commercialisé par Fluka ;
- EDC supporté par un polymère commercialisé par Fluka (code 09657) avec un taux de fonctionnalisation de 1,4 mmol d'EDC par gramme de polymère ;
- NHS : N-hydroxysulfosuccinimide de sodium commercialisé par Fluka. A partir de ces réactifs, les solutions suivantes ont été réalisées :

- Solution $A_1$ : Solution aqueuse à 10 % en pigment 1.
- Solution $A_2$ : Solution aqueuse à 10 % en pigment 2.
- Solution B : Solution aqueuse à 10 % en polyvinylamine / n-vinylformamide (Catiofast VFH commercialisée par BASF).
- Solution C : Solution aqueuse contenant 0,023 g d'EDC et 0,026 g de NHS.
- Solution $D_1$ : Solution aqueuse à 10 % en pigment 1 et à 10 % en polyvinylamine / n-vinylformamide (Catiofast VFH commercialisée par BASF).
- Solution $D_2$: Solution aqueuse à 10 % en pigment 2 et à 10 % en polyvinylamine / n-vinylformamide (Catiofast VFH commercialisée par BASF).
- Solution E : Solution aqueuse contenant 0,085 g d'EDC supporté sur polymère et 0,026 g de NHS.

**Exemple 1 :**

1) 0,5 g de la solution $A_1$ est appliqué sur une mèche de cheveux naturels propres et secs. La mèche est ensuite séchée au casque.
2) 0,5 g de la solution B est ensuite appliqué sur la mèche sèche. Sans séchage intermédiaire, 0,3 g de la solution C est appliqué.
3) La mèche est placée 30 minutes au casque.
4) La mèche est rincée et shampouinée.

Le même mode opératoire est mis en oeuvre pour la solution $A_2$.
Avec la solution $A_1$, la mèche obtenue est très colorée en rose. Avec la solution $A_2$, la mèche obtenue est très colorée en rouge.
Les mèches sont ensuite soumises à un test de résistance aux shampooings.
La couleur des cheveux est mesurée à l'aide d'un spectrocolorimètre MINOLTA CM3600d® dans le système CIELab. Dans ce système, L* représente la clarté, a* la teinte et b* la saturation.
La dégradation de la couleur après six shampooings est estimée selon la formule suivante :

$$\%dégradation = 100 * \frac{DE\ 6Shamp}{DE\ Tém}$$

avec :

$$DE\ tém = \sqrt{(a*tém - a*BN)^2 + (b*tém - b*BN)^2 + (L*tém - L*BN)^2}$$

et

$$DE\ 6Shamp = \sqrt{(a*tém - a*6Sh)^2 + (b*tém - b*6Sh)^2 + (L*tém - L*6Sh)^2}$$

dans lesquelles a*BN, b*BN et L*BN sont les valeurs de a*, b* et L* de la mèche non colorée, a*tém, b*tém et L*tem les valeurs de a*, b* et L* de la mèche colorée avant shampooing, tandis que a*6Sh, b*6Sh et L*6Sh les valeurs de a*, b* et L* de la mèche colorée après 6 shampooings.

Avec la solution $A_1$, la dégradation de la couleur est de 7,91 % après 6 shampooings.

Avec la solution $A_2$, la dégradation de la couleur est de 26,92 % après 6 shampooings.

Ces résultats montrent que la coloration obtenue avec le procédé conforme à l'invention présente une bonne résistance aux shampooings.

### Exemple 2 :

1) 0,5 g de la solution $D_1$ est appliqué sur cheveux propres et secs. Sans séchage intermédiaire, 0,3 g de la solution C est appliqué.
2) La mèche est placée 30 minutes au casque.
3) La mèche est rincée et shampouinée.

Le même mode opératoire est mis en oeuvre pour la solution $D_2$.

Avec la solution $D_1$, la mèche obtenue est très colorée en rose. Avec la solution $D_2$, la mèche obtenue est très colorée en rouge.

### Exemple 3 :

1) 0,5 g de la solution $A_1$ est appliqué sur cheveux propres et secs. La mèche est ensuite séchée au casque.
2) 0,5 g de la solution B est ensuite appliqué sur la mèche sèche. Sans séchage intermédiaire, 0,3 g de la solution C est appliqué.
3) La mèche est ensuite séchée au fer à lisser à 180 °C.
4) La mèche est rincée et shampouinée.

Le même mode opératoire est mis en oeuvre pour la solution $A_2$.

Avec la solution $A_1$, la mèche obtenue est très colorée en rose. Avec la solution $A_2$, la mèche obtenue est très colorée en rouge.

### Exemple 4 :

1) 0,5 g de la solution $A_1$ est appliqué sur cheveux propres et secs. La mèche est ensuite séchée au casque.
2) 0,3 g de la solution C est ensuite appliqué sur la mèche sèche. Sans séchage intermédiaire, 0,5 g de la solution B est appliqué.
3) La mèche est placée 30 minutes au casque.
4) La mèche est rincée et shampouinée.

Le même mode opératoire est mis en oeuvre pour la solution $A_2$.

Avec la solution $A_1$, la mèche obtenue est très colorée en rose. Avec la solution $A_2$, la mèche obtenue est très colorée en rouge.

### Exemple 5 :

1) 0,5 g de la solution $A_1$ est appliqué sur cheveux propres et secs. La mèche est ensuite séchée au casque.
2) 0,5 g de la solution B est ensuite appliqué sur la mèche sèche. Sans séchage intermédiaire, 0,3 g de la solution E est appliqué.
3) La mèche est placée 30 minutes au casque.
4) La mèche est rincée et shampouinée.

Le même mode opératoire est mis en oeuvre pour la solution $A_2$.

Avec la solution $A_1$, la mèche obtenue est très colorée en rose. Avec la solution $A_2$, la mèche obtenue est très colorée en rouge.

Des résultats comparables à ceux qui sont exposés ci-dessus sont obtenus avec les polyamines suivantes :

- ε-polylysine (commercialisée par Chisso) ;
- polyéthylèneimine (Lupasol P commercialisée par BASF) ;
- poly(allylamine hydrochloride) (commercialisée par Aldrich, code 28322-3).

## Revendications

1. Composition pour la coloration des fibres kératiniques comprenant, dans un milieu cosmétiquement acceptable :

   - au moins un composé portant au moins une fonction amine ayant un poids moléculaire supérieur à 300 g.mol$^{-1}$
   - au moins un pigment différent de l'oxyde de fer, de l'oxyde de titane et de la silice ; et
   - au moins un agent de couplage chimique pour la formation de liaisons amide.

2. Composition selon la revendication 1, dans laquelle le ou les composés portant au moins une fonction amine ont un poids moléculaire supérieur à 1000 g.mol$^{-1}$.

3. Composition selon la revendication 1 ou 2, dans laquelle le ou les composés portant au moins une fonction amine sont des polymères.

4. Composition selon la revendication 3, dans laquelle le ou les polymères sont des polyamines.

5. Composition selon la revendication 4, dans laquelle la ou les polyamines sont choisies parmi la polyvinylamine, la polyéthylèneamine, la polylysine, le chitosane, la polyallylamine, l'aminodextrane, l'amino cellulose, l'aminopolyvinylacétal.

6. Composition selon l'une quelconque des revendications précédentes, dans laquelle le ou les pigments sont sous forme de poudre ou de pâte pigmentaire.

7. Composition selon la revendication 6, dans laquelle au moins un pigment est un pigment minéral choisi parmi les oxydes de zirconium ou de cérium, les oxydes de chrome, le violet de manganèse, le bleu outremer, l'hydrate de chrome et le bleu ferrique.

8. Composition selon la revendication 6, dans laquelle au moins un pigment est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

9. Composition selon la revendication 6, dans laquelle au moins un pigment est un pigment composite composé de particules comportant:

   - un noyau inorganique.
   - au moins un liant assurant la fixation des pigments organiques sur le noyau, et
   - au moins un pigment organique recouvrant au moins partiellement le noyau.

10. Composition selon la revendication 6, dans laquelle au moins un pigment est une laque constituée par un substrat inorganique choisi parmi l'alumine, la silice, le borosilicate de calcium et de sodium ou le borosilicate de calcium et d'aluminium, et l'aluminium sur lequel est adsorbé un colorant.

11. Composition selon la revendication 6, dans laquelle au moins un pigment est un pigment à effets spéciaux choisi parmi les pigments nacrés, les pigments à effets interférentiels non fixés sur un substrat, les pigments fluorescents, les pigments phosphorescents, les pigments photochromiques, les pigments thermochromiques et les quantum dots.

12. Composition selon la revendication 11, dans laquelle le ou les pigments nacrés sont choisis parmi le mica recouvert de titane, ou d'oxychlorure de bismuth, le mica recouvert de titane et d'oxydes de fer, le mica recouvert de titane et de bleu ferrique ou d'oxyde de chrome, le mica recouvert de titane et d'un pigment organique tel que défini à la revendication 8, les pigments nacrés à base d'oxychlorure de bismuth.

13. Composition selon la revendication 11, dans laquelle le ou les pigments à effet interférentiel non fixés sur un substrat sont choisis parmi les cristaux liquides, les paillettes holographiques interférentielles.

14. Composition selon l'une quelconque des revendications précédentes, dans laquelle le ou les pigments sont des pigments colorés.

15. Composition selon l'une quelconque des revendications précédentes, dans laquelle le ou les pigments sont en dispersion.

16. Composition selon l'une quelconque des revendications précédentes, dans laquelle le ou les agents de couplage chimiques sont choisis parmi les dérivés du carbodiimide; le 1,1'-carbonyldiimidazole; les sels de phosphonium ; les phosphonates ; les phosphoramides ; les dérivés de dialcoxytriazine.

17. Composition selon la revendication 16, dans laquelle le ou les dérivés du carbodiimide sont choisis parmi les composés de formule (I) suivante :

$$R_1\text{-}N=C=N\text{-}R_2 \qquad (I)$$

dans laquelle $R_1$ et $R_2$ désignent, indépendamment l'un de l'autre:

- un atome d'hydrogène;
- un radical alkyle non substitué ou substitué par un ou plusieurs substituants choisis parmi :

  • un radical hydroxyle;
  • un radical amino ;
  • un radical mono ou dialkylamino;
  • un radical mono ou di(mono ou polyhydroxyalkyl)amino;
  • un radical aryle ;
  • un groupement hétérocyclique;

- un radical aryle;
- un radical arylalkyle.

18. Composition selon la revendication 17, dans laquelle $R_1$ et $R_2$ ne désignent pas un atome hydrogène.

19. Composition selon la revendication 18, dans laquelle $R_1$ et $R_2$ désignent un radical alkyle non substitué ou substitué.

20. Composition selon l'une quelconque des revendications 16 à 19, dans laquelle le ou les dérivés du carbodiimide sont choisis parmi le N-(3-diméthylaminopropyl) N'-(éthyl) carbodiimide ; le N,N'-dicyclohexylcarbodiimide.

21. Composition selon l'une quelconque des revendications 16 à 20, dans laquelle le ou les dérivés du carbodiimide sont solubles dans l'eau ou dans un solvant alcoolique.

22. Composition selon l'une quelconque des revendications précédentes, dans laquelle le ou les composés portant au moins une fonction amine sont chacun présents à des concentrations comprises entre 0,1 et 60 % en poids par rapport au poids total de la composition.

23. Composition selon l'une quelconque des revendications précédentes, dans laquelle le ou les pigments sont chacun présents à des concentrations comprises entre 0,05 et 80 % en poids par rapport au poids total de la composition.

24. Composition selon l'une quelconque des revendications précédentes, dans laquelle le ou les agents de couplage chimiques sont chacun présents à des concentrations comprises entre 0,05 et 30 % en poids par rapport au poids total de la composition.

25. Composition selon l'une quelconque des revendications précédentes, comprenant au moins un composé additionnel qui, en association avec le ou tes agents de couplage chimiques, sont capables de favoriser la formation de liaisons amide.

26. Composition selon la revendication 25, dans laquelle le ou les composés additionnels sont des composés portant au moins une fonction N-hydroxy.

27. Composition selon la revendication 26, dans laquelle le ou les composés portant au moins une fonction N-hydroxy sont choisis parmi le N-hydroxysuccinimide; le N-hydroxysulfosuccinimide.

28. Composition selon la revendication 26 ou 27, dans laquelle le ou les composés portant au moins une fonction N-hydroxy contiennent de plus au moins un groupement polaire.

29. Composition selon l'une quelconque des revendications 25 à 28, dans laquelle le ou les composés additionnels sont chacun présents à des concentrations comprises entre 0,05 et 30 % en poids par rapport au poids total de la composition.

30. Procédé de coloration des fibres kératiniques, **caractérisé par le fait que** l'on applique sur les fibres kératiniques:

   - au moins un composé portant au moins une fonction amine tel que défini à l'une quelconque des revendications 1 à 5;
   - au moins un pigment tel que défini à l'une quelconque des revendication 1 et 6 à 14; et
   - au moins un agent de couplage chimique tel que défini à l'une quelconque des revendications 1 et 16 à 21.

31. Procédé selon la revendication 30, dans lequel le ou les composés portant au moins une fonction amine, le ou les pigments et le ou les agents de couplage chimiques sont appliqués sur les fibres kératiniques à partir de plusieurs compositions contenant le ou les composés portant au moins une fonction amine, le ou les pigments et le ou les agents de couplage chimiques, seuls ou en mélange, ou à partir d'une seule composition contenant le ou les composés portant au moins une fonction amine, le ou les pigments et le ou les agents de couplage chimiques.

32. Procédé selon la revendication 31, dans lequel la composition comprenant le ou les pigments est appliquée avant la ou les compositions comprenant le ou les composés portant au moins une fonction amine et / ou le ou les agents de couplage chimiques.

33. Procédé selon l'une quelconque des revendications 30 à 32, dans lequel on applique sur les fibres kératiniques au moins un composé additionnel tel que défini à l'une quelconque des revendications 25 à 28 pour favoriser la formation de liaisons amide.

34. Dispositif à plusieurs compartiments, **caractérisé par le fait qu'**il contient au moins deux compositions telles que l'ensemble desdites compositions comprend :

   - au moins un composé portant au moins une fonction amine tel que défini à l'une quelconque des revendications 1 à 5;
   - au moins un pigment tel que défini à l'une quelconque des revendications 1 et 6 à 14; et
   - au moins un agent de couplage chimique tel que défini à l'une quelconque des revendications 1 et 16 à 23.

35. Dispositif selon la revendication 34, dans lequel l'ensemble desdites compositions comprend de plus au moins un composé additionnel tel que défini à l'une quelconque des revendications 25 à 28.

36. Utilisation pour la coloration des fibres kératiniques d'au moins un composé portant au moins une fonction amine tel que défini à l'une quelconque des revendications 1 à 5, d'au moins un pigment tel que défini à l'une quelconque des revendication 1 et 6 à 14 et d'au moins un agent de couplage chimique tel que défini à l'une quelconque des revendications 1 et 16 à 21.

37. Utilisation selon la revendication 36, dans laquelle la coloration des fibres kératiniques est résistante aux shampooings.

**EP 1 600 148 B1**

**Claims**

1. Composition for dyeing keratin fibres, comprising, in a cosmetically acceptable medium:

   - at least one compound bearing at least one amine function having a molecular weight of greater than 300 g.mol$^{-1}$;
   - at least one pigment other than iron oxide, titanium oxide and silica; and
   - at least one chemical coupling agent for the formation of amide bonds.

2. Composition according to Claim 1, in which the compound(s) bearing at least one amine function has (have) a molecular weight of greater than 1000 g.mol$^{-1}$.

3. Composition according to Claim 1 or 2, in which the compound(s) bearing at least one amine function is (are) polymers.

4. Composition according to Claim 3, in which the polymer(s) is (are) polyamines.

5. Composition according to Claim 4, in which the polyamine(s) is (are) chosen from polyvinylamine, polyethyleneamine, polylysine, chitosan, polyallylamine, aminodextran, aminocellulose and aminopolyvinyl acetal.

6. Composition according to any one of the preceding claims, in which the pigment(s) is (are) in the form of powder or of pigmentary paste.

7. Composition according to Claim 6, in which at least one pigment is a mineral pigment chosen from zirconium oxide or cerium oxide, chromium oxides, manganese violet, ultramarine blue, chromium hydrate and ferric blue.

8. Composition according to Claim 6, in which at least one pigment is an organic pigment chosen from nitroso, nitro, azo, xanthene, quinoline, anthraquinone and phthalocyanin compounds, compounds of metallic complex type, and isoindolinone, isoindoline, quinacridone, perinone, perylene, diketopyrrolopyrrole, thioindigo, dioxazine, triphenylmethane and quinophthalone compounds.

9. Composition according to Claim 6, in which at least one pigment is a composite pigment composed of particles comprising:

   - a mineral core,
   - at least one binder for fixing the organic pigments to the core, and
   - at least one organic pigment at least partially covering the core.

10. Composition according to Claim 6, in which at least one pigment is a lake constituted of a mineral substrate chosen from alumina, silica, calcium sodium borosilicate or calcium aluminium borosilicate, and aluminium on which is adsorbed a dye.

11. Composition according to Claim 6, in which at least one pigment is a pigment with special effects chosen from nacreous pigments, pigments with interference effects not bound to a substrate, fluorescent pigments, phosphorescent pigments, photochromic pigments, thermochromic pigments and quantum dots.

12. Composition according to Claim 11, in which the nacreous pigment(s) is (are) chosen from mica coated with titanium or with bismuth oxychloride, mica coated with titanium and with iron oxides, mica coated with titanium and with ferric blue or chromium oxide, mica coated with titanium and with an organic pigment as defined in Claim 8, and nacreous pigments based on bismuth oxychloride.

13. Composition according to Claim 11, in which the pigment(s) with an interference effect not bound to a substrate is (are) chosen from liquid crystals and holographic interference flakes.

14. Composition according to any one of the preceding claims, in which the pigment(s) is (are) coloured pigments.

15. Composition according to any one of the preceding claims, in which the pigment(s) is (are) in dispersion.

16. Composition according to any one of the preceding claims, in which the chemical coupling agent(s) is (are) chosen from carbodiimide derivatives; 1,1'-carbonyldiimidazole; phosphonium salts; phosphonates; phosphoramides; dialkoxytriazine derivatives.

17. Composition according to Claim 16, in which the carbodiimide derivative(s) is (are) chosen from the compounds of formula (I) below:

$$R_1\text{-}N\text{=}C\text{=}N\text{-}R_2 \qquad (I)$$

in which $R_1$ and $R_2$ denote, independently of each other:

- a hydrogen atom;
- an alkyl radical, which is unsubstituted or substituted with one or more substituents chosen from:

  • a hydroxyl radical;
  • an amino radical;
  • a monoalkylamino or dialkylamino radical;
  • a mono- or di(mono- or polyhydroxyalkyl)amino radical;
  • an aryl radical;
  • a heterocyclic group;

- an aryl radical;
- an arylalkyl radical.

18. Composition according to Claim 17, in which $R_1$ and $R_2$ do not denote a hydrogen atom.

19. Composition according to Claim 18, in which $R_1$ and $R_2$ denote an unsubstituted or substituted alkyl radical.

20. Composition according to any one of Claims 16 to 19, in which the carbodiimide derivative(s) is (are) chosen from N-(3-dimethylaminopropyl)-N'-(ethyl)carbodiimide; N,N'-dicyclohexylcarbodiimide.

21. Composition according to any one of Claims 16 to 20, in which the carbodiimide derivative(s) is (are) soluble in water or in an alcoholic solvent.

22. Composition according to any one of the preceding claims, in which the compound(s) bearing at least one amine function is (are) each present in concentrations of between 0.1% and 60% by weight relative to the total weight of the composition.

23. Composition according to any one of the preceding claims, in which the pigment(s) is (are) each present in concentrations of between 0.05% and 80% by weight relative to the total weight of the composition.

24. Composition according to any one of the preceding claims, in which the chemical coupling agent(s) is (are) each present in concentrations of between 0.05% and 30% by weight relative to the total weight of the composition.

25. Composition according to any one of the preceding claims, comprising at least one additional compound, which, in combination with the chemical coupling agent(s), is capable of promoting the formation of amide bonds.

26. Composition according to Claim 25, in which the additional compound(s) is (are) compounds bearing at least one N-hydroxyl function.

27. Composition according to Claim 26, in which the compound(s) bearing at least one N-hydroxyl function is (are) chosen from N-hydroxysuccinimide; N-hydroxysulfosuccinimide.

28. Composition according to Claim 26 or 27, in which the compound(s) bearing at least one N-hydroxyl function also contain(s) at least one polar group.

29. Composition according to any one of Claims 25 to 28, in which the additional compound(s) is (are) each present in concentrations of between 0.05% and 30% by weight relative to the total weight of the composition.

30. Process for dyeing keratin fibres, **characterized in that** the following are applied to the keratin fibres:

- at least one compound bearing at least one amine function as defined in any one of Claims 1 to 5;
- at least one pigment as defined in any one of Claims 1 and 6 to 14; and
- at least one chemical coupling agent as defined in any one of Claims 1 and 16 to 21.

**31.** Process according to Claim 30, in which the compound(s) bearing at least one amine function, the pigment(s) and the chemical coupling agent(s) are applied to the keratin fibres using several compositions containing the compound (s) bearing at least one amine function, the pigment(s) and the chemical coupling agent(s), alone or as a mixture, or using only one composition containing the compound(s) bearing at least one amine function, the pigment(s) and the chemical coupling agent(s).

**32.** Process according to Claim 31, in which the composition comprising the pigment(s) is applied before the composition (s) comprising the compound(s) bearing at least one amine function and/or the chemical coupling agent(s).

**33.** Process according to any one of Claims 30 to 32 in which at least one additional compound as defined in any one of Claims 25 to 28 is applied to the keratin fibres to promote the formation of amide bonds.

**34.** Multi-compartment device, **characterized in that** it contains at least two compositions such that the combination of the said compositions comprises:

- at least one compound bearing at least one amine function as defined in any one of Claims 1 to 5;
- at least one pigment as defined in any one of Claims 1 and 6 to 14; and
- at least one chemical coupling agent as defined in any one of Claims 1 and 16 to 23.

**35.** Device according to Claim 34, in which the combination of the said compositions also comprises at least one additional compound as defined in any one of Claims 25 to 28.

**36.** Use, for dyeing keratin fibres, of at least one compound bearing at least one amine function as defined in any one of Claims 1 to 5, of at least one pigment as defined in any one of Claims 1 and 6 to 14 and of at least one chemical coupling agent as defined in any one of Claims 1 and 16 to 21.

**37.** Use according to Claim 36, in which the coloration of the keratin fibres is shampoo-fast.

**Patentansprüche**

**1.** Zusammensetzung zum Färben von Keratinfasern, die in einem kosmetisch akzeptablen Medium enthält:

- mindestens eine Verbindung, die mindestens eine Aminofunktion aufweist, mit einer Molmasse über 300 $g \cdot mol^{-1}$,
- mindestens ein Pigment, das von Eisenoxid, Titanoxid und Siliciumoxid verschieden ist; und
- mindestens ein chemisches Kupplungsmittel für die Bildung von Amidbindungen.

**2.** Zusammensetzung nach Anspruch 1, wobei die Verbindung(en) mit mindestens einer Aminofunktion einer Molmasse über 1 000 $g \cdot mol^{-1}$ aufweisen.

**3.** Zusammensetzung nach Anspruch 1 oder 2, wobei die Verbindung(en) mit mindestens einer Aminofunktion Polymere sind.

**4.** Zusammensetzung nach Anspruch 3, wobei das oder die Polymere Polyamine sind.

**5.** Zusammensetzung nach Anspruch 4, wobei das oder die Polyamine unter Polyvinylamin, Polyethylenamin, Polylysin, Chitosan, Polyallylamin, Aminodextran, Aminocellulose und Aminopolyvinylacetal ausgewählt sind.

**6.** Zusammensetzung nach einem der vorhergehenden Ansprüche, wobei das oder die Pigmente in Pulverform oder als Pigmentpaste vorliegen.

**7.** Zusammensetzung nach Anspruch 6, wobei mindestens ein Pigment ein anorganisches Pigment ist, das unter den Oxiden von Zirconium oder Cer, den Oxiden von Chrom, Manganviolett, Ultramarinblau, Chromhydrat und Eisenblau ausgewählt ist.

**8.** Zusammensetzung nach Anspruch 6, wobei mindestens ein Pigment ein organisches Pigment ist, das unter den Nitrosoverbindungen, Nitroverbindungen, Azoverbindungen, Xanthenverbindungen, Chinolinverbindungen, Anthrachinonverbindungen, Phthalocyaninverbindungen, Verbindungen vom Typ der Metallkomplexe, Isoindolinonverbindungen, Isoindolinverbindungen, Chinacridonverbindungen, Perinonverbindungen, Perylenverbindungen, Diacetopyrrolopyrrolverbindungen, Thioindigoverbindungen, Dioxazinverbindungen, Triphenylmethanverbindungen und Chinophthalonverbindungen ausgewählt ist.

**9.** Zusammensetzung nach Anspruch 6, wobei mindestens ein Pigment ein Verbundpigment ist, das aus Partikeln besteht, die aufweisen:

- einen anorganischen Kern,
- mindestens ein Bindemittel, das die Fixierung der organischen Pigmente an dem Kern sicherstellt, und
- mindestens ein organisches Pigment, das den Kern zumindest zum Teil bedeckt.

**10.** Zusammensetzung nach Anspruch 6, wobei mindestens ein Pigment ein Lack ist, der aus einem anorganischen Substrat besteht, das unter Aluminiumoxid, Siliciumoxid, Calcium und Natriumborsilicat, Calcium und Aluminiumborsilicat und Aluminium ausgewählt ist, an dem ein Farbmittel adsorbiert ist.

**11.** Zusammensetzung nach Anspruch 6, wobei mindestens ein Pigment ein Pigment mit Spezialeffekten ist, das unter den Perlglanzpigmenten, Pigmenten mit Interferenzeffekten, die nicht an einem Substrat fixiert sind, fluoreszierenden Pigmenten, phosphoreszierenden Pigmenten, photochromen Pigmenten, thermochromen Pigmenten und Quantum Dots ausgewählt ist.

**12.** Zusammensetzung ach Anspruch 11, wobei die Perlglanzpigmente unter den mit Titan oder Bismutoxidchlorid bedeckten Glimmer-Pigmenten, mit Eisenoxiden bedeckten Titan-Glimmer-Pigmenten, mit Eisenblau oder Chromoxid bedeckten Titan-Glimmer-Pigmenten, mit einem organischen Pigment, wie es in Anspruch 8 definiert ist, bedeckten Titan-Glimmer-Pigmenten und Perlglanzpigmenten auf der Basis von Bismutoxidchlorid ausgewählt sind.

**13.** Zusammensetzung nach Anspruch 11, wobei das oder die Pigmente mit Interferenzeffekt, die nicht auf einem Substrat fixiert sind, unter den Flüssigkristallen und interferierenden holographischen Pailletten ausgewählt sind.

**14.** Zusammensetzung nach einem der vorhergehenden Ansprüche, wobei das oder die Pigmente farbige Pigmente sind.

**15.** Zusammensetzung nach einem der vorhergehenden Ansprüche, wobei das oder die Pigmente dispergiert sind.

**16.** Zusammensetzung nach einem der vorhergehenden Ansprüche, wobei das oder die chemischen Kupplungsmittel unter den Carbodiimidderivaten; 1,1'-Carbonyldiimidazol; Phosphoniumsalzen; Phosphonaten; Phorphoramiden; Dialkoxytriazinderivaten ausgewählt sind.

**17.** Zusammensetzung nach Anspruch 16, wobei das oder die Carbodiimidderivate unter den Verbindungen der folgenden Formel (I) ausgewählt sind:

$$R_1\text{-}N\text{=}C\text{=}N\text{-}R_2 \qquad (I),$$

worin $R_1$ und $R_2$ unabhängig voneinander bedeuten:

- ein Wasserstoffatom;
- eine Alkylgruppe, die unsubstituiert vorliegt oder mit einem oder mehreren Substituenten substituiert ist, die ausgewählt sind unter:

  • Hydroxy;
  • Amino;
  • Mono- oder Dialkylamino;
  • Mono- oder Di(mono- oder polyhydroxyalkyl)amino;
  • Aryl;

• einer heterocyclischen Gruppe;

- eine Arylgruppe;
- eine Alkylarylgruppe.

18. Zusammensetzung nach Anspruch 17, wobei $R_1$ und $R_2$ kein Wasserstoffatom bedeuten.

19. Zusammensetzung nach Anspruch 18, wobei $R_1$ und $R_2$ eine substituierte oder unsubstituierte Alkylgruppe bedeuten.

20. Zusammensetzung nach einem der Ansprüche 16 bis 19, wobei das oder die Carbodiimidderivate unter N-(3-Dimethylaminopropyl)-N'-(ethyl)-carbodiimid; N,N'-Dicyclohexylcarbodiimid ausgewählt sind.

21. Zusammensetzung nach einem der Ansprüche 16 bis 20, wobei das oder die Carbodiimidderivate in Wasser oder einem alkoholischen Lösungsmittel löslich sind.

22. Zusammensetzung nach einem der vorhergehenden Ansprüche, wobei die Verbindung(en) mit mindestens einer Aminofunktion jeweils in Konzentrationen von 0,1 bis 60 Gew.-%, bezogen auf das Gesamtgewicht der Zusammensetzung, enthalten sind.

23. Zusammensetzung nach einem der vorhergehenden Ansprüche, wobei das oder die Pigmente jeweils in Konzentrationen von 0,05 bis 80 Gew.-%, bezogen auf das Gesamtgewicht der Zusammensetzung, enthalten sind.

24. Zusammensetzung nach einem der vorhergehenden Ansprüche, wobei das oder die chemischen Kupplungsmittel jeweils in Konzentrationen von 0,05 bis 30 Gew.-%, bezogen auf das Gesamtgewicht der Zusammensetzung, enthalten sind.

25. Zusammensetzung nach einem der vorhergehenden Ansprüche, die mindestens eine ergänzende Verbindung enthält, die in Kombination mit dem oder den chemischen Kupplungsmitteln befähigt ist, die Bildung der Amidbindung zu fördern.

26. Zusammensetzung nach Anspruch 25, wobei die ergänzende(n) Verbindung(en) Verbindungen mit mindestens einer N-Hydroxyfunktion sind.

27. Zusammensetzung nach Anspruch 26, wobei die Verbindung(en) mit mindestens einer N-Hydroxyfunktion unter N-Hydroxysuccinimid; N-Hydroxysulfosuccinimid ausgewählt sind.

28. Zusammensetzung nach Anspruch 26 oder 27, wobei die Verbindung(en) mit mindestens einer N-Hydroxyfunktion ferner mindestens eine polare Gruppe aufweisen.

29. Zusammensetzung nach einem der Ansprüche 25 bis 28, wobei die ergänzende(n) Verbindung(en) jeweils in Konzentrationen von 0,05 bis 30 Gew.-%, bezogen auf das Gesamtgewicht der Zusammensetzung, enthalten sind.

30. Verfahren zum Färben von Keratinfasern, **dadurch gekennzeichnet, dass** auf die Fasern aufgebracht werden:

- mindestens eine Verbindung, die mindestens eine Aminofunktion aufweist, wie sie in einem der Ansprüche 1 bis 5 definiert ist;
- mindestens ein Pigment, wie es in einem der Ansprüche 1 und 6 bis 14 definiert ist; und
- mindestens ein chemisches Kupplungsmittel, wie es in einem der Ansprüche 1 und 16 bis 21 definiert ist.

31. Verfahren nach Anspruch 30, wobei die Verbindung(en) mit mindestens einer Aminofunktion, das oder die Pigmente und das oder die chemischen Kupplungsmittel auf die Keratinfasern ausgehend von mehreren Zusammensetzungen, die die Verbindung(en) mit mindestens einer Aminofunktion, das oder die Pigmente und das oder die chemischen Kupplungsmittel einzeln oder im Gemisch enthalten, oder ausgehend von einer einzigen Zusammensetzung aufgetragen werden, die die Verbindung(en) mit mindestens einer Aminofunktion, das oder die Pigmente und das oder die chemischem Kupplungsmittel enthält.

32. Verfahren nach Anspruch 31, wobei die Zusammensetzung, die das oder die Pigmente enthält, vor der oder den Zusammensetzung(en) aufgebracht wird, die die Verbindung(en) mit mindestens einer Aminogruppe und/oder das oder die chemischen Kupplungsmittel enthält (enthalten).

33. Verfahren nach einem der Ansprüche 30 bis 32, wobei auf die Keratinfasern mindestens eine ergänzende Verbindung aufgebracht wird, wie sie in einem der Ansprüche 25 bis 28 definiert ist, um die Bildung der Amidbindung zu begünstigen.

34. Vorrichtung mit mehreren Abteilungen, **dadurch gekennzeichnet, dass** sie mindestens zwei Zusammensetzungen enthält, die so sind, dass alle Zusammensetzungen enthalten:

- mindestens eine Verbindung, die mindestens eine Aminofunktion aufweist, wie sie in einem der Ansprüche 1 bis 5 definiert ist;
- mindestens ein Pigment, wie es in einem der Ansprüche 1 und 6 bis 14 definiert ist; und
- mindestens ein chemisches Kupplungsmittel, wie es in einem der Ansprüche und 16 bis 23 definiert ist.

35. Vorrichtung nach Anspruch 34, wobei die gesamten Zusammensetzungen ferner mindestens eine ergänzende Verbindung enthalten, wie sie in einem der Ansprüche 25 bis 28 definiert ist.

36. Verwendung mindestens einer Verbindung, die mindestens eine Aminofunktion aufweist, wie sie in einem der Ansprüche 1 bis 5 definiert ist, mindestens eines Pigments, wie es in einem der Ansprüche 1 und 6 bis 14 definiert ist, und mindestens eines chemischen Kupplungsmittels, wie es in einem der Ansprüche 1 und 16 bis 21 definiert wurde, zum Färben von Keratinfasern.

37. Verwendung nach Anspruch 36, wobei die Färbung der Keratinfasern gegenüber Haarwäschen beständig ist.

**EP 1 600 148 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2741530
- FR 2679771
- EP 1184426 A
- US 6225198 B
- US 5990479 A
- EP 1184426 A2

**Littérature non-brevet citée dans la description**

- **DABBOUSSI B.O. et al.** CdSe)ZnS core-shell quantum dots : synthesis and characterisation of a size series of highly luminescent nanocristallites. *Journal of physical chemistry B,* 1997, vol. 101, 9463-9475
- **PENG, XIAOGANG et al.** Epitaxial Growth of highly Luminescent CdSe/CdS core/shell nanocrystals with photostability and electronic accessibility. *Journal of the American Chemical Society,* vol. 119 (30), 7019-7029